# EUROPEAN PATENT SPECIFICATION

(11) **EP 3 592 244 B1**
(45) Date of publication and mention of the grant of the patent: **08.07.2026**
(21) Application number: 18710457.5
(22) Date of filing: 09.03.2018
(51) Int. Cl.: A61B 10/00, A61B 10/02, A61B 17/00

(54) **A SELF-SAMPLING DEVICE FOR VAGINAL FLUID COLLECTION**
EIGENPROBENAHMEVORRICHTUNG ZUR SAMMLUNG VON VAGINALFLÜSSIGKEIT
DISPOSITIF D'AUTO-ÉCHANTILLONNAGE POUR LA COLLECTE DE FLUIDE VAGINAL

(30) Priority: 10.03.2017 SE 1750273
(43) Date of publication of application: 15.01.2020
(73) Proprietor: Aprovix AB, 169 70 Solna (SE)
(72) Inventor: ANDERSSON, Henry, 754 19 Uppsala (SE); NYGREN, Sören, 184 70 Åkersberga (SE); WILANDER, Erik, 793 70 Tällberg (SE)
(74) Representative: AWA Sweden AB
(86) International application number: PCT/EP2018/055918
(87) International publication number: WO 2018/162716

(56) References cited:
- EP-A1- 2 263 552
- WO-A1-2014/189130
- US-A- 5 295 952
- US-A1- 2009 012 424
- US-A1- 2012 310 113
- US-A1- 2015 230 872

## Description

### Technical field

The present invention relates generally to the field of screening and health control. More specifically the invention relates to self-sampling devices for vaginal fluid testing to detect for example virus-associated cervical cancer, microbial infections and pathological changes.

### Background

Pap smear, cervical smear, or smear test are different names of a method of cervical screening used to detect potentially pre-cancerous and cancerous processes in the cervix. Abnormal findings are often followed up by more sensitive diagnostic procedures, and, if warranted, interventions that aim to prevent progression to cervical cancer. A common type of laboratory analysis made on sample materia taken from the vagina is DNA analysis for HPV (human papillomavirus). Traditionally, cervical smear testing requires scraping of a woman's cervix with a sampling device, such as a spatula or a brush. This sampling is generally performed by medical professionals like gynecologists, midwifes or nurses in a clinical environment. Many women, who now refrain from such gynecological testing, would participate if the sampling could be carried out at home and/or by the women themselves. Self and home sampling would therefore increase the participation in the screening, and by that means, decrease the incidence of cervical cancer. US 2003/0028123 A1 discloses an intra-vaginal self-administered cell collection device, but no snap-fit connection. US 5,445,164 A discloses a quick-release connection in the context of a cervical tissue sampling device, but the release mechanism is based on sliding a sleeve rearward along shaft until engaged tab and portions are exposed. The pin may then be manually disengaged from aperture to complete detachment of stem from shaft.

US 2015/230872 A1 discloses a sampling system with a flexible shaft having a handle at one end, wherein the shaft is configured to allow an individual to self collect a cell sample from mucous tissue, and a sample collecting element removably connectable with the other end of the shaft and operable to collect a cell sample from a mucous tissue of an individual. Nothing is, however, disclosed about how rotation of the shaft relatively a hole configured to receive the shaft exposes differently shaped projected profiles, such that when in a specific position, the projected profile fits within the bounds of a correspondingly projected profile exposed by a passage of an interlocking means, and such that when not in the specific position, the projected profile of the shaft does not fit within the bounds of the projected profile exposed by the passage.

US 2012/310113 A1 discloses a fluid sample collection system, to facilitate collection of biological specimens and particularly to facilitate collection of saliva, urine and stool specimens from human or animal species for the purpose of extracting purified DNA and/or RNA.

EP 2263552 A1 discloses cell collection device comprising means for collecting cells and a longitudinal handle whereby the means for collecting cells is attachable to the handle using a snap release joint.

US 2009/012424 A1 discloses a device for removing and smearing cells for a cytological examination with a handle at whose front end a device for the collection of the cells is arranged. The device is embodied as a cone tapering towards the front in which a stabilizing device acting on a longitudinal extension of the cone is arranged.

US 5295952 A discloses a swab for use in laparoscopy, comprising an outer, generally tubular shank with a handle or gripping end and a working end. The inner shaft is slidably received in the shank and has a working end and a gripping end. A changeable, disposable absorbent tip is operably connected to the working end of the shaft.

WO 2014/189130 A1 discloses a cell collection device comprising a tube body, a cell collection member which can be mounted on a tip of the tube body, a lock part which holds the cell collection member mounted to the tip of the tube body, and an operational member which changes the position of the lock part to a locked position or a released position.

In addition to the above, sampling systems are also in demand for DNA analysis. Law enforcement officials, paternity agents, etc. are constantly taking DNA samples to help solve crimes, determine paternity, etc. As the results of the tests done on these samples dramatically affect people's lives and may be desired as evidence in legal proceedings, the sampling must be done in a manner in which the sample contamination is reduced or avoided.

One example of a system suitable for self-testing is described in European Patent EP1903946B1 to Aprovix AB, Sweden. This known system brings many advantages over prior art solutions.

The sample collection element is removably connectable with the shaft by a snap-fit connection which is formed by protrusions at an end of the sample collection means and adapted to snap-fit in grooves contained in extensions at the other end of the shaft. The snap-fit connection with protrusions and grooves is dependent on exact fit between protrusions and grooves in order to perform consistently for holding the sample collection element connected to the handle and also allow for detachment of the sample collection element from the handle when the sample collection element is within the sealable unit. Such exact fit requires extensive quality systems with measurement of produced parts (i.e. handle and sample collection element). Testing is time-consuming and expensive and so is discarding batches of produced parts.

During sampling with the self-sampling device shown in EP1903946B1 there is a slight risk that excessive side forces brings the snap locking engagement between sample collection body and handle out of engagement, resulting in losing control of the sample collection body at sampling. Further, the protrusions are released from the grooves contained in the extension by rotation of the sample collection element. However, the protrusions often tend to grip the recesses also after rotation, at least to some extent, wherein easy disengagement is prevented.

Hence, there is the need of an improved self-sampling device performing consistently without requiring extensive quality testing in production.

### Summary

An object of the invention is to provide an improved self-sampling device which mitigates the drawbacks discussed above.

According to a first aspect of the invention, this and other objects are achieved by a self-sampling device for vaginal fluid from a vagina of a user, said self-sampling device comprising an elongate first body for insertion into the vagina of the user, and a sample collection body for collecting a vaginal fluid sample from the user. The sample collection body is attachable to an inner end portion of the elongate first body by a releasable connection means. The elongate first body is configured such that it is operable by the user to collect a fluid sample from the vagina with the sample collection body attached to the first body. The releasable connection means comprises a first interlocking means provided on one of the first body and the sample collection body, and a second interlocking means provided on the other one of the elongate first body and the sample collection body. The first interlocking means is provided with a shaft defining a rotational axis substantially transversal to a longitudinal axis of the first body. The second interlocking means comprises a first hole or recess configured to receive the shaft upon connection of the first and second interlocking means for rotation of the sample collection body relative to the first body about the rotational axis between a first position, in which the longitudinal axis of the sample collection body is aligned with the longitudinal axis of the first body, and a second position in which the longitudinal axis of the sample collection body is rotated about the rotational axis away from said first position a predetermined distance. Rotation of the shaft relative to the hole exposes differently shaped projected profiles, such that when in the second position, the projected profile fits within the bounds of a correspondingly projected profile exposed by a passage of the second interlocking means, and such that when not in the second position, the projected profile of the shaft does not fit within the bounds of the projected profile exposed by the passage. The first and second interlocking means are further provided with first and second respective corresponding snap-locking means configured to releasably engage each other when in the first position to prevent relative rotation between the first and second interlocking means when the snap-locking means are engaged. The first and second interlocking means are configured such that in said second position the first and second interlocking means are disengageable by movement of the shaft out of the first hole or recess in a predetermined disengagement direction. Also, the first and second interlocking means are configured such that in said first position the shaft is confined within said first hole or recess.

The shaft and corresponding hole or recess provides for well-defined relative rotational movement between sample collection body and first body. The snap-locking means functions to hold the sample collection body in the first position during use, enabling controlled movement of the sample collection body by holding an outer end portion of the first body and operating the sample collection body for sampling, just like the use described in EP1903946B1 mentioned above. The shaft of the first attachment means limits the degrees of freedom available for the sample collection body to move, so that it can only move from its first position according to said well-defined rotational movement. Thus, the snap-locking means needs mainly to cope with momentum applied to the sample collection body and needs not to cope with relative translational movement. Such combination of shaft and snap-locking means provides for lower risk of losing control of the sample collection body at sampling. The design even enables use of a weaker snap-lock mechanism with sustained control of the sample collection body in the vagina. Using a weaker mechanism provides for use of less material and thereby less environmental impact. After sampling, the sample collection body is withdrawn from the vagina and inserted into a small container for protection of the sample. After insertion, momentum is applied to rotate the sample collection body and first body into the second position. In said second position, the first body is simply moved away from the sample collection body such that the sample collection body is left in the container by gravitational forces acting on the sample collection body. The first body is then discarded and the container sealed, for example by a lid, ready for future analysis.

Since the first and second interlocking means are disengageable in said second position, but not in said first position, a firm hold is provided in the first position, and easy disengagement is provided in said second position. The sample collection body can easily be separated from said handle with a minimum of force and without need of manually holding the sample collection body to pull it off the first body. Also, the sample collection body is well controlled during sampling.

In an embodiment, the shaft is provided at a first proximal portion of the first interlocking means. Further, the first one of the corresponding snap-locking means is provided at a first distal portion of the first interlocking means.

The proximal portion of the respective interlocking means is the portion that is closest to the sample collection body or first body on which it is provided. By providing the shaft at the proximal portion of the first attachment means, there is little distance between shaft and the sample collection body or first body on which it is provided, and hence the shaft is rigidly attached and cannot move much. This provides for better control of the relative rotation/movement between the first body and the sample collection body. Likewise, the distal positioning of the first snap-locking means provides for a greater distance between snap-locking means and the sample collection body or first body to which the first interlocking means is attached, and thus enables increased flexibility of the snap-locking means. Increased flexibility allows for design of a snap-locking mechanism where a main source of biasing force is the first interlocking means itself, thereby providing for a simple and robust design.

To further specify the location of the proximal and distal portions, the first proximal portion of the first interlocking means is adjacent the first body or sample collection body on which the first interlocking means is provided. Likewise, the first distal portion is further away from the sample collection body or first body on which the first interlocking means is provided than the first proximal portion.

In an embodiment, the first hole or recess is laterally open such that the shaft is moveable laterally out of the first hole or recess from said second position at disengagement.

Making the first hole or recess laterally open enables the shaft to be moved laterally/radially out of the first hole or recess rather than having to be moved along the longitudinal axis of the shaft.

In an embodiment, the first interlocking means comprises a forked body comprising opposite shanks together defining an intermediate space extending along the longitudinal axis of the first body. The shaft extends from one shank to the other across said intermediate space. The second interlocking means comprises an elongate stem adapted to fit in the intermediate space between the shanks. Also, the first hole or recess of the second interlocking means is laterally open for receiving the shaft.

Making the first hole or recess laterally open enables the shaft to be moved laterally/radially out of the first hole or recess rather than having to be moved along the longitudinal axis of the shaft. This in turn allows for the shaft to be fixed at both ends without hindering disengagement of the first and second interlocking means. Fixing the shaft at both ends provides a robust fixation of the shaft, thereby enabling use of weaker shaft with less material. Using less material is of advantage, since the self-sampling device is a single-use device and thus cost and environmental impact is of more importance that long-term durability.

In an embodiment, the first hole or recess is laterally open in a direction along the length of the second interlocking means.

Such configuration of the first hole or recess enables easy disengagement of the sample collection body once it is positioned vertically in the container and the first member rotated to bring the sample collection body to the second position. Since the shaft is moveable vertically out of the first hole or recess and the sample collection body is thus free to fall down into the container by the force of gravity.

In an embodiment, the shaft is mainly cylindrical with at least one recessed portion along the length of the shaft. Also, the first hole or recess has a cross sectional shape with a circular mid portion corresponding to the main diameter of the shaft. Said mid portion opens laterally outwards in the form of a passage with a smallest width less than the diameter of the shaft. Further, the recessed portion(s) of the shaft defines a projected profile of the shaft in a plane through the longitudinal axis of the shaft, wherein said projected profile is smaller than the passage such that the shaft is movable through the passage from said second position.

By providing the shaft with such recessed portions, the shaft is prevented from moving through the path except for when the shaft is oriented in the second position in which the shaft exposes its projected profile smaller than the passage. This allows the first and second interlocking means to engage to always allow relative rotation whilst at the same time preventing relative translation when not in said second position, thereby confining the shaft within the first hole or recess.

In an embodiment, the passage widens outwardly away from the circular mid portion. Such widening configuration of the passage provides surfaces for guiding the shaft into the passage at assembly of the shaft. Thereby making both manual and automatic assembly easier and more predictive.

In an embodiment, the first hole or recess comprises an auxiliary recessed portion extending inwards from the circular mid portion along the length of the second interlocking means, away from the passage and partly towards the second snap-locking means. The width of the auxiliary recessed portion is less than the diameter of the mid portion.

The auxiliary recessed portion extending further inwards improves the resiliency of the second connection means such that the shaft can be forced into the circular mid portion even though not in said second position. This in turn enables quick and easy assembly of first body and the sample collection body. Another advantage is that the improved resiliency enables controlled biasing between the shaft and the first hole or recess, should a biasing engagement be of interest.

In an embodiment, the corresponding snap-locking means are provided in the interface between stem and shanks.

Providing the snap-locking means in the interface between shanks and stem enables the resilience of the shanks to force the corresponding snap-locking means together, such as by forcing one or more protrusions into one or more corresponding recesses. This provides an easy means of achieving a desired biasing functionality of the snap-locking means.

In an embodiment, protrusions of the first snap-locking means are provided on the shanks. Further, the at least one recess of the snap-locking means is provided in the stem. Also, the protrusions of the first snap-locking means comprise forwardly chamfered portions oriented for the stem to gradually push the protrusions apart upon insertion of the stem between the chamfered portions along the longitudinal axis of the first body.

This configuration of the snap-locking means promotes quick and easy assembly of first body at insertion of the stem along the longitudinal direction of the first body into the intermediate space, wherein the risk of damaging the protrusions during assembly is lowered since the chamfered portions make it easier for the stem to push apart the protrusions of the snap-locking means during assembly and thereby reduces the stress on the protrusions. Deformed or damaged surfaces on the protrusions of the snap-locking means would increase the risk of malfunction of the self-sampling device, such as too loose snap-functionality or direct risk of losing control of the sample collection body at sampling.

**Table of reference numerals**

| | | | |
|---|---|---|---|
| 1 | self-sampling device | 18 | longitudinal axis of sample collecting body |
| 2 | first body | 19 | disengagement direction |
| 3 | sample collection body | 20 | intermediate space |
| 4 | inner end portion | 21 | stem |
| 5 | releasable connection means | 22 | passage |
| 6 | first interlocking means | 23 | mid portion |
| 7 | second interlocking means | 24 | recessed portions |
| 8 | shaft | 25 | auxiliary portion |
| 9 | rotational axis | 26 | container |
| 10 | longitudinal axis of first body | 27 | rotational axis defined by hole or recess |
| 11 | first hole or recess | 28 | outer end portion |
| 12 | first snap-locking means | d1 | smallest width of passage |
| 13 | second snap-locking means | d2 | projected profile width of shaft |
| 14 | first proximal portion | d3 | main diameter of shaft |
| 15 | first distal portion | D | predetermined distance |
| 16 | first shank | P | projection plane |
| 17 | second shank | | |

### Brief description of drawings

Figs. 1-18 all show a first embodiment of the invention.
Figs. 1-7 show views in which the sample collection body is rotated to its second position, ready for disengagement from the rest of the self-sampling device.
Figs. 8-15 show views in which the sample collection body is rotated to its first position ready for sampling.

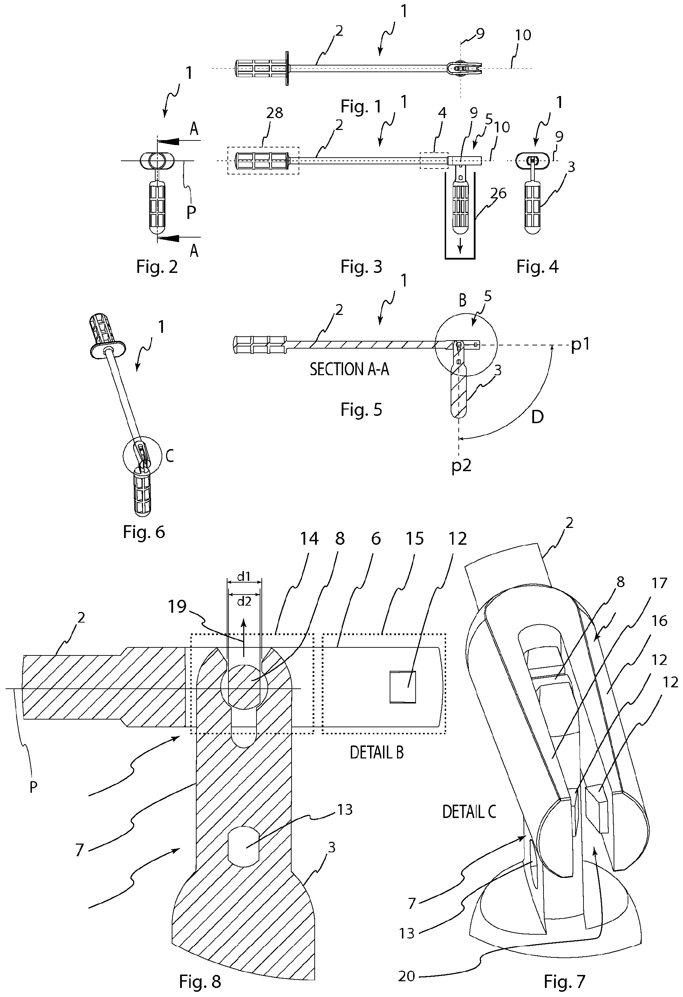

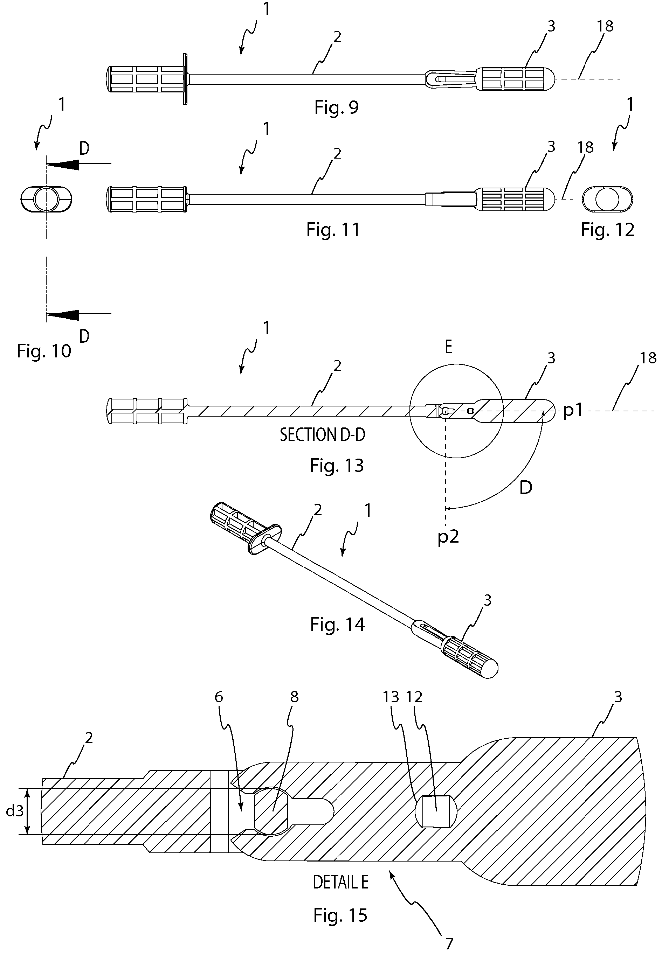

Figs. 1 and 9 are top views.
Figs. 2 and 10 are back views.
Figs. 3 and 11 are side views.
Figs. 4 and 12 are front views.
Figs. 5 and 13 are sectional side views.
Figs. 6 and 14 are diagonal perspective views from above.
Figs. 7, 8 and 15 are enlarged views of the first and second interlocking means.

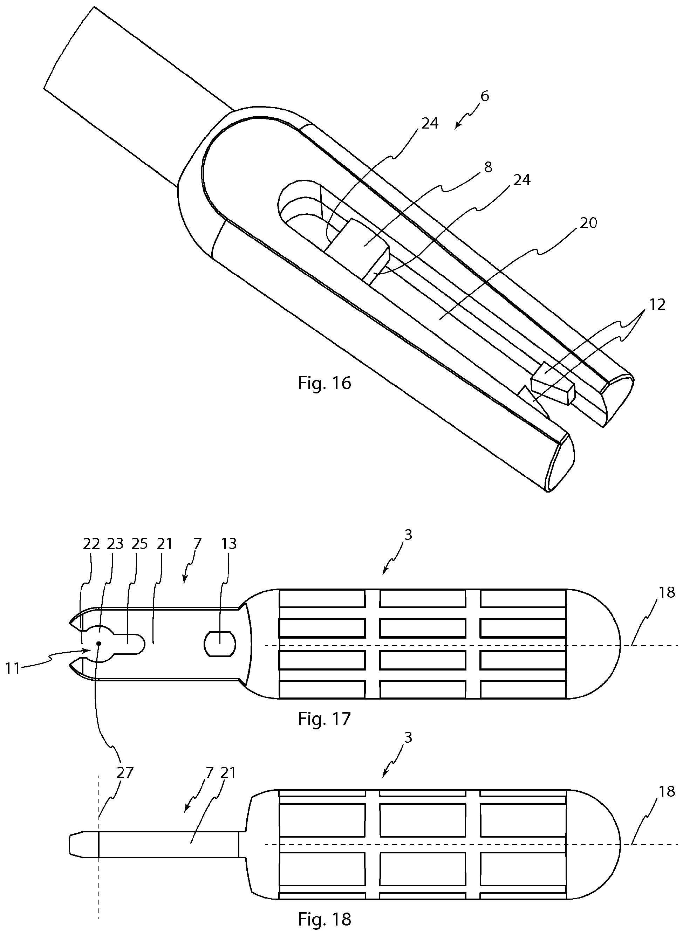

Fig. 16 is an enlarged detail view showing the first interlocking means.
Fig. 17 is a side view of the sample collection body and the second interlocking means provided on the sample collection body.
Fig. 18 is a top view of the sample collection body and the second interlocking means provided on the sample collection body also shown in Fig. 17.

### Detailed description

The disclosed embodiments will hereinafter be described in more detail with reference to the accompanying drawings in which some embodiments of the invention are shown. This invention may, however, be embodied in many different forms and should not be construed as limited to the embodiments set forth herein. Rather, these embodiments are provided by way of example so that this disclosure will be thorough and complete, and will fully convey the scope of the invention to those skilled in the art. Like numbers refer to like elements throughout.

The self-sampling device of the present invention represents a further development of the self-sampling device described in EP1903946B1 and it is to be used in the same manner for collecting a sample and sending it for analysis by mail in a dedicated small container/sealable unit. Therefore, this type of use of the self-sampling device is known and the present description will focus on the new improved design of the self-sampling device itself. At sampling, the self-sampling device operated to rotate the sample collection body around its longitudinal axis while pushing it laterally towards the mucous membranes of the vagina such that vaginal fluid is stuck in recesses of the sample collection body.

Hence, the present invention provides a self-sampling device 1 which may be employed by an individual to easily and reliably, at home or at a visit to a medical location, under hygienic conditions, take a vaginal fluid sample intended for subsequent laboratory analysis, for example, but not limited to, DNA analysis for HPV. The samples can be transported in a small sealable container without risk of contamination or transmission of infective agents, and thereafter be analyzed by chemical methods, such as DNA analysis, or by other immunological or microbiological methods.

With reference to Figs. 1-18, a first embodiment of the self-sampling device 1 according to the invention will be described in the following. The self-sampling device 1 comprises an elongate first body for insertion into the vagina of the user, and a sample collection body 3 for collecting a vaginal fluid sample from the user. The sample collection body 3 is attachable to an inner end portion 4 of the elongate first body by a releasable connection means 5. The inner end portion is the portion of the first body that in use is inserted into the vagina, whereas an outer end portion of the first body is a portion that in use is held by the user operating the self-sampling device. The first body is thus used as a handle.

The elongate first body is configured such that it is operable by the user to collect a vaginal fluid sample from the vagina with the sample collection body 3 attached to the first body. This implies that the first body is of suitable length and shape to be handled by an individual for performing self-sampling in the vagina. In the first embodiment, the length of the first elongate body is 145 mm wherein the narrow cylindrical body portion is 93 mm long and has a diameter of 4mm, and wherein the gripping portion has a width/diameter of 10 mm and a length of 32 mm. The rest of the first elongate-body is occupied by a first interlocking means 6 as described below.

The releasable connection means 5 comprises a first interlocking means 6 provided on the elongate first body and a second interlocking means 7 provided on the sample collection body 3.

The first interlocking means 6 comprises a forked body comprising opposite shanks 16, 17 together defining an intermediate space 20 extending along the longitudinal axis 10 of the first body. A shaft 8 extends from one shank 16 to the other 17 across said intermediate space 20 for defining a rotational axis 9 substantially transversal to a longitudinal axis 10 of the first body. Both opposite ends of the shaft 8 are thus attached/supported.

The second interlocking means 7 comprises an elongate stem 21 adapted to fit in the intermediate space 20 between the shanks 16, 17. The stem 21 is provided with a through hole configured to receive the shaft 8 upon connection of the first 6 and second 7 interlocking means for rotation of the sample collection body 3 relative to the first body about the rotational axis 9 between a first position, in which the longitudinal axis 18 of the sample collection body 3 is aligned with the longitudinal axis of sample collection body 3 as shown in Fig. 9-15, and a second position in which the longitudinal axis 10 of the sample collection body 3 is rotated about the rotational axis 9 away from said first position a predetermined distance D, as shown in Figs. 1-8.

The first 6 and second 7 interlocking means are further provided with first and second respective corresponding snap-locking means configured to releasably engage each other when in the first position to prevent relative rotation between the first 6 and second 7 interlocking means when the snap-locking means are engaged. The corresponding snap-locking means 12, 13 are provided in the interface between stem 21 and shanks 16, 17 with opposite protrusions 12 extending inwards into the intermediate space 20 between the shanks 16, 17, and with the stem 21being provided with a through recess 13 corresponding to said opposite protrusions 12.

The first 6 and second 7 interlocking means are configured such that in said second position the first 6 and second 7 interlocking means are disengageable by movement of the shaft 8 out of the hole 11 in a predetermined disengagement direction 19. Also, the first 6 and second 7 interlocking means are configured such that in said first position the shaft 8 is confined within said first hole 11.

The first hole 11 of the second interlocking means 7 is laterally open in a direction along the length of the second interlocking means 7. Such configuration of the first hole or recess enables easy disengagement of the sample collection body once it is positioned vertically in the container and the first member rotated to bring the sample collection body to the second position. Since the shaft is moveable vertically out of the first hole or recess and the sample collection body is thus free to fall down into the container by the force of gravity. It should be understood that within the context of this description 'laterally open' means that the hole 11 is open in a direction perpendicular to a rotational axis defined by the hole 11, for example in a radial direction should the hole 11 be of cylindrical or conical shape. The rotational axis defined by the hole 11 corresponds to the rotational axis 9 defined by the shaft 8, when the first 6 and second 7 interlocking means are connected. Here it should be understood that the hole 11 could take other forms than cylindrical, as long as the hole 11 fits to the shaft 8 to support the sample element for rotation about the rotational axis 9. Generally, the hole 11 needs to exhibit at least three supporting surfaces for being distributed around the shaft 8 to control the rotational movement about the shaft 8.

The shaft 8 is mainly cylindrical with two recessed portions 24 extending along the length of the shaft 8, said portions being provided on opposite sides of the longitudinal axis of the shaft 8. The hole 11 has a cross sectional shape with a circular mid portion 23 corresponding to the main diameter of the shaft 8. As illustrated in Fig. 7, the main diameter d3 is to be understood as being the diameter of the shaft 8 without regard to the recessed portions 24. The opposite recessed portions 24 are planar, but could in other embodiments have other shapes, such as curved in one or two directions. The circular mid portion 23 could have other shapes, as long as it functions to support the second interlocking means 7 for rotation about the shaft 8.

A shown in Figs. 7 and 15, the mid portion 23 opens outwardly in the form of a passage 22 with a smallest width d1 less than the main diameter d3 (See fig. 15) of the shaft 8.

The recessed portions 24 of the shaft 8 define a projected profile of the shaft 8 in a plane P through the longitudinal axis of the shaft 8 normal to the disengagement direction 19. The projected profile is smaller than the passage 22 such that the shaft 8 is movable through the passage 22 from said second position, for example having a projected profile width d2 smaller than the smallest width d1 of the passage 22.

It should be understood that many shapes of the shaft 8 and of the passage 22 are possible within the scope of the present invention, and that the important teaching is that rotation of the shaft 8 relative to the hole 11 exposes differently shaped projected profiles, such that in a specific position (i.e. the second position), the projected profile fits within the bounds of a correspondingly projected profile exposed by the passage 22 of the second interlocking means 7, and such that in other positions, the projected profile of the shaft 8 does not fit within the bounds of the projected profile exposed by the passage 22. Thus, the shaft 8 is confined within the hole 11 except for in the second position where it is movable straight out of the hole 11. This enable controlled relative rotation between first 6 and second 7 interlocking means 7 within a predetermined first operational range (not in the second position, but between the second position and the first position), whilst also enabling free detachment of the first 6 and second 7 interlocking means when in another operational range outside the first operational range (i.e. in the second position). The configuration of the first embodiment depicted in Figs. 1-15 is advantageous since it is robust and easy to produce using relatively simple injection molding tools.

The passage 22 widens outwardly away from the circular mid portion 23. This makes it easier to move the shaft 8 into the passage 22. Also, the hole 11 comprises an auxiliary recessed portion 24 extending inwards from the mid portion 23 along the length of the second interlocking means 7, away from the passage 22 and partly towards the second snap-locking means 13, the width of the auxiliary recessed portion 25 being less than the diameter of the mid portion 23.

In other embodiments, the stem 21 and shanks 16, 17 could switch positions, such that the shanks 16, 17 are provided on the sample collection body and the stem 21 on the first body.

The recesses of the sample collection body should be able to absorb mucous fluid and cells therein, and maintain these materials in place during retraction of the self-sampling device 1. In the present embodiment, the size of the sample collection body is slightly bigger than the one described in EP1903946B1, in order to allow for collection of more sample material and hold it deeper in recesses of the sample collection body.

The first body and the sample collection body can be manufactured of any suitable materials as desired. For example, these components may be formed of the same or different plastic materials. In the first embodiment, the first body is made of a plastic material, such as polypropylene, with a flexural modulus giving the first body flexibility to follow the anatomy of the vagina to reach portio vaginalis and fornix vaginalis and at the same time rigid enough to get a close contact between the sample collection body and the mucous tissue ectocervix.

A suitable method of manufacturing the first body and the sample collection body is injection molding, since it provides good repeatability and low cost.

At use, the self-sampling device 1 is moved to its first position with the sample collection body attached to the first body/handle/shaft 8. Thereafter, the sample collection body is inserted into the vaginal region and moved to collect a sample. Thereafter, the self-sampling device 1 is retracted and the sample collection body inserted in a sealable transportation container 26. Subsequently, the first body is rotated by one hand relative to the sample collection body to the second position. The sample collection body is prevented from rotating by the container which is held by the other hand. By keeping the container upright, the sample collection body is free to fall deeper into the container.

## Claims

1. A self-sampling device (1) for vaginal fluid from a vagina of a user, said self-sampling device (1) comprising
an elongate first body (2) for insertion into the vagina of the user, and
a sample collection body (3) for collecting a vaginal fluid sample from the user,
wherein the sample collection body (3) is attachable to an inner end portion (4) of the elongate first body (2) by a releasable connection means (5),
wherein the elongate first body (2) is configured such that it is operable by the user to collect a fluid sample from the vagina with the sample collection body (3) attached to the first body (2),
wherein said releasable connection means (5) comprises a first interlocking means (6) provided on one of the first body (2) and the sample collection body (3), and a second interlocking means (7) provided on the other one of the elongate first body (2) and the sample collection body (3),
wherein the first interlocking means (6) is provided with a shaft (8) defining a rotational axis (9) substantially transversal to a longitudinal axis (10) of the first body (2),
wherein the second interlocking means (7) comprises a first hole or recess (11) configured to receive the shaft (8) upon connection of the first (6) and second (7) interlocking means for rotation of the sample collection body (3) relative to the first body (2) about the rotational axis (9) between a first position (p1), in which the longitudinal axis (18) of the sample collection body (3) is aligned with the longitudinal axis (10) of the first body (2), and a second position (p2) in which the longitudinal axis (18) of the sample collection body (3) is rotated about the rotational axis (9) away from said first position (p1) a predetermined distance (D),
wherein rotation of the shaft (8) relative to the hole (11) exposes differently shaped projected profiles, such that when in the second position (p2), the projected profile fits within the bounds of a correspondingly projected profile exposed by a passage (22) of the second interlocking means (7), and such that when not in the second position (p2), the projected profile of the shaft (8) does not fit within the bounds of the projected profile exposed by the passage (22),
wherein the first (6) and second (7) interlocking means are further provided with first (12) and second (13) respective corresponding snap-locking means configured to releasably engage each other (12), (13) when in the first position (p1) to prevent relative rotation between the first (6) and second (7) interlocking means when the snap-locking means (12, 13) are engaged,
wherein the first (6) and second (7) interlocking means are configured such that in said second position (p2) the first (6) and second (7) interlocking means are disengageable by movement of the shaft (8) out of the first hole or recess (11) in a predetermined disengagement direction (19), and wherein the shaft (8) and the first hole or recess (11) are configured such that in said first position (p1) the sample collection body (3) is prevented from disengaging from the inner end portion (4) of the elongate first body (2) as the projected profile of the shaft does not fit within the bounds of the projected profile exposed by the passage.

2. A self-sampling device (1) according to claim 1, wherein the shaft (8) is provided at a first proximal portion (14) of the first interlocking means (6), wherein the first one (12) of the corresponding snap-locking means (12, 13) is provided at a first distal portion (15) of the first interlocking means (6).

3. A self-sampling device (1) according to claim 2, wherein the first proximal portion (14) of the first interlocking means (6) is adjacent the first body (2) or sample collection body (3) on which the first interlocking means (6) is provided, and wherein the first distal portion is further away from the sample collection body or first body on which the first interlocking means is provided than the first proximal portion.

4. A self-sampling device (1) according to any one of claims 1-3, wherein the first hole or recess (11) is laterally open such that the shaft (8) is moveable laterally out of the first hole or recess (11) from said second position (p2) at disengagement.

5. A self-sampling device (1) according to claim 1, wherein the first interlocking means (6) comprises a forked body comprising opposite shanks (16, 17) together defining an intermediate space (20) extending along the longitudinal axis (10) of the first body (2),
wherein said shaft (8) extends from one shank (16) to the other (17) across said intermediate space (20), wherein the second interlocking means (7) comprises an elongate stem (21) adapted to fit in the intermediate space (20) between the shanks (16, 17), and
wherein said first hole or recess (11) of the second interlocking means (7) is laterally open for receiving the shaft (8).

6. A self-sampling device (1) according to claim 5, wherein the shaft (8) is provided at a first proximal portion (14) of the first interlocking means (6), wherein a first one (12) of the corresponding snap-locking means (12, 13) is provided at a first distal portion (15) of the first interlocking means (6).

7. A self-sampling device (1) according to anyone of claims 5 or 6 wherein the first hole or recess (11) is laterally open in a direction along the length of the second interlocking means (7).

8. A self-sampling device (1) according to claim 7,
wherein the shaft (8) is mainly cylindrical with at least one recessed portion (24) along the length of the shaft (8), and
wherein the first hole or recess (11) has a cross sectional shape with a circular mid portion (23) corresponding to the main diameter of the shaft (8),
wherein said mid portion (23) opens laterally outwards in the form of the passage (22) with a smallest width less than the diameter of the shaft (8), and wherein the recessed portion(s) (24) of the shaft (8) defines a projected profile of the shaft (8) in a plane through the longitudinal axis of the shaft (8), said projected profile being smaller than the passage (22) such that the shaft (8) is movable through the passage (22) from said second position (p2).

9. A self-sampling device (1) according to claim 8, wherein the passage (22) widens outwardly away from the circular mid portion (23).

10. A self-sampling device (1) according to any one of the claims 7-9, wherein the first hole or recess (11) comprises an auxiliary recessed portion (25) extending inwards from the circular mid portion (23) along the length of the second interlocking means (7), away from the passage (22) and partly towards the second snap-locking means (13), the width of the auxiliary recessed portion (25) being less than the diameter of the mid portion (23).

11. A self-sampling device (1) according to any one of claims 7-10, wherein the corresponding snap-locking means (12, 13) are provided in the interface between stem (21) and shanks (16, 17).

12. A self-sampling device (1) according to claim 11, wherein protrusions (12) of the first snap-locking means (12) are provided on the shanks (16, 17), wherein at least one recess of the second snap-locking means (13) is provided in the stem (21), and wherein the protrusions (13) of the first snap-locking means (12) comprise forwardly chamfered portions oriented for the stem (21) to gradually push the protrusions (13) apart upon insertion of the stem (21) between the chamfered portions.

13. A system comprising a self-sampling device (1) according to any one of the preceding claims, a sealable container (26) for storing the sample collection body (3) after use.

## Patentansprüche

1. Vorrichtung (1) zur Selbstentnahme einer Vaginalsekretprobe aus der Vagina einer Benutzerin, wobei die Vorrichtung (1) zur Selbstentnahme von Proben Folgendes umfasst:
einen länglichen ersten Körper (2) zum Einführen in die Vagina der Benutzerin und
einen Probensammelkörper (3) zum Sammeln einer Vaginalsekretprobe von der Benutzerin,
wobei der Probensammelkörper (3) durch ein lösbares Verbindungsmittel (5) an einem inneren Endabschnitt (4) des länglichen ersten Körpers (2) anbringbar ist,
wobei der längliche erste Körper (2) so ausgestaltet ist, dass er durch die Benutzerin betreibbar ist, um mit dem an dem ersten Körper (2) angebrachten Probensammelkörper (3) eine Sekretprobe aus der Vagina zu sammeln,
wobei das lösbare Verbindungsmittel (5) ein erstes Verriegelungsmittel (6), das an dem ersten Körper (2) oder dem Probensammelkörper (3) vorgesehen ist, und ein zweites Verriegelungsmittel (7), das an dem jeweils anderen des länglichen ersten Körpers (2) und des Probensammelkörpers (3) vorgesehen ist, umfasst,
wobei das erste Verriegelungsmittel (6) mit einer Welle (8) versehen ist, die eine Drehachse (9) definiert, die im Wesentlichen quer zu einer Längsachse (10) des ersten Körpers (2) verläuft,
wobei das zweite Verriegelungsmittel (7) ein erstes Loch oder eine erste Aussparung (11) umfasst, die dazu ausgestaltet ist, die Welle (8) bei Verbindung des ersten (6) und des zweiten (7) Verriegelungsmittels zum Drehen des Probensammelkörpers (3) relativ zu dem ersten Körper (2) um die Drehachse (9) zwischen einer ersten Position (p1), in der die Längsachse (18) des Probensammelkörpers (3) auf die Längsachse (10) des ersten Körpers (2) ausgerichtet ist, und einer zweiten Position (p2), in der die Längsachse (18) des Probensammelkörpers (3) um die Drehachse (9) um eine vorbestimmte Strecke (D) von der ersten Position (p1) weg gedreht wird, aufzunehmen,
wobei durch eine Drehung der Welle (8) relativ zu dem Loch (11) unterschiedlich geformte projizierte Profile freigelegt werden, so dass das projizierte Profil, wenn es sich in der zweiten Position (p2) befindet, innerhalb der Grenzen eines entsprechend projizierten, durch einen Durchgang (22) des zweiten Verriegelungsmittels (7) freigelegten Profils liegt, und so dass das projizierte Profil der Welle (8), wenn es sich nicht in der zweiten Position (p2) befindet, nicht innerhalb der Grenzen des projizierten durch den Durchgang (22) freigelegten Profils liegt,
wobei das erste (6) und das zweite (7) Verriegelungsmittel ferner mit einem ersten (12) und einem zweiten (13) entsprechenden Schnappverriegelungsmittel versehen sind, die dazu ausgestaltet sind, lösbar miteinander in Eingriff zu treten (12), (13), wenn sie sich in der ersten Position (p1) befinden, um eine Relativdrehung zwischen dem ersten (6) und dem zweiten (7) Verriegelungsmittel zu verhindern, wenn die Schnappverriegelungsmittel (12, 13) in Eingriff sind,
wobei das erste (6) und das zweite (7) Verriegelungsmittel so ausgestaltet sind, dass das erste (6) und das zweite (7) Verriegelungsmittel in der zweiten Position (p2) durch Bewegung der Welle (8) aus dem ersten Loch oder der ersten Aussparung (11) in einer vorbestimmten Ausrückrichtung (19) ausrückbar sind, und wobei die Welle (8) und das erste Loch oder die erste Aussparung (11) so ausgestaltet sind, dass in der ersten Position (p1) verhindert wird, dass der Probensammelkörper (3) aus dem inneren Endabschnitt (4) des länglichen ersten Körpers (2) ausrückt, da das projizierte Profil der Welle nicht innerhalb der Grenzen des durch den Durchgang freigelegten projizierten Profils liegt.

2. Vorrichtung (1) zur Selbstentnahme von Proben nach Anspruch 1, wobei die Welle (8) an einem ersten proximalen Abschnitt (14) des ersten Verriegelungsmittels (6) vorgesehen ist, wobei das erste (12) der entsprechenden Schnappverriegelungsmittel (12, 13) an einem ersten distalen Abschnitt (15) des ersten Verriegelungsmittels (6) vorgesehen ist.

3. Vorrichtung (1) zur Selbstentnahme von Proben nach Anspruch 2, wobei der erste proximale Abschnitt (14) des ersten Verriegelungsmittels (6) dem ersten Körper (2) oder dem Probensammelkörper (3), an dem das erste Verriegelungsmittel (6) vorgesehen ist, benachbart ist und wobei der erste distale Abschnitt weiter von dem Probensammelkörper oder dem ersten Körper, an dem das erste Verriegelungsmittel vorgesehen ist, entfernt ist als der erste proximale Abschnitt.

4. Vorrichtung (1) zur Selbstentnahme von Proben nach einem der Ansprüche 1 - 3, wobei das erste Loch oder die erste Aussparung (11) seitlich offen ist, so dass die Welle (8) beim Ausrücken seitlich aus dem ersten Loch oder der ersten Aussparung (11) aus der zweiten Position (p2) heraus bewegbar ist.

5. Vorrichtung (1) zur Selbstentnahme von Proben nach Anspruch 1, wobei das erste Verriegelungsmittel (6) einen gabelförmigen Körper umfasst, der gegenüberliegende Schenkel (16, 17) umfasst, die zusammen einen Zwischenraum (20) definieren, der sich entlang der Längsachse (10) des ersten Körpers (2) erstreckt,
wobei sich die Welle (8) über den Zwischenraum (20) von einem Schenkel (16) zu dem anderen (17) erstreckt, wobei das zweite Verriegelungsmittel (7) einen länglichen Schaft (21) umfasst, der dazu ausgeführt ist, in den Zwischenraum (20) zwischen den Schenkeln (16, 17) zu passen, und
wobei das erste Loch oder die erste Aussparung (11) des zweiten Verriegelungsmittels (7) zur Aufnahme der Welle (8) seitlich offen ist.

6. Vorrichtung (1) zur Selbstentnahme von Proben nach Anspruch 5, wobei die Welle (8) an einem ersten proximalen Abschnitt (14) des ersten Verriegelungsmittels (6) vorgesehen ist, wobei ein erstes (12) der entsprechenden Schnappverriegelungsmittel (12, 13) an einem ersten distalen Abschnitt (15) des ersten Verriegelungsmittels (6) vorgesehen ist.

7. Vorrichtung (1) zur Selbstentnahme von Proben nach einem der Ansprüche 5 oder 6, wobei das erste Loch oder die erste Aussparung (11) in einer entlang der Länge des zweiten Verriegelungsmittels (7) verlaufenden Richtung seitlich offen ist.

8. Vorrichtung (1) zur Selbstentnahme von Proben nach Anspruch 7,
wobei die Welle (8) hauptsächlich zylindrisch ist und mindestens einen ausgesparten Abschnitt (24) entlang der Länge der Welle (8) aufweist und
wobei das erste Loch oder die erste Vertiefung (11) eine Querschnittsform mit einem kreisförmigen Mittelabschnitt (23) aufweist, der dem Hauptdurchmesser der Welle (8) entspricht,
wobei sich der Mittelabschnitt (23) in Form des Durchgangs (22) seitlich nach außen öffnet, wobei eine kleinste Breite kleiner als der Durchmesser der Welle (8) ist, und wobei der/die ausgesparte/n Abschnitt/e (24) der Welle (8) ein projiziertes Profil der Welle (8) in einer Ebene durch die Längsachse der Welle (8) definiert/definieren, wobei das projizierte Profil kleiner als der Durchgang (22) ist, so dass die Welle (8) aus der zweiten Position (p2) durch den Durchgang (22) bewegbar ist.

9. Vorrichtung (1) zur Selbstentnahme von Proben nach Anspruch 8, wobei sich der Durchgang (22) von dem kreisförmigen Mittelabschnitt (23) weg nach außen erweitert.

10. Vorrichtung (1) zur Selbstentnahme von Proben nach einem der Ansprüche 7 - 9, wobei das erste Loch oder die erste Aussparung (11) einen ausgesparten Hilfsabschnitt (25) umfasst, der sich von dem kreisförmigen Mittelabschnitt (23) entlang der Länge des zweiten Verriegelungsmittels (7) nach innen, weg von dem Durchgang (22) und teilweise zu dem zweiten Schnappverriegelungsmittel (13) hin erstreckt, wobei die Breite des ausgesparten Hilfsabschnitts (25) kleiner als der Durchmesser des Mittelabschnitts (23) ist.

11. Vorrichtung (1) zur Selbstentnahme von Proben nach einem der Ansprüche 7 - 10, wobei die entsprechenden Schnappverriegelungsmittel (12, 13) in der Schnittstelle zwischen dem Schaft (21) und den Schenkeln (16, 17) vorgesehen sind.

12. Vorrichtung (1) zur Selbstentnahme von Proben nach Anspruch 11, wobei Vorsprünge (12) des ersten Schnappverriegelungsmittels (12) an den Schenkeln (16, 17) vorgesehen sind, wobei mindestens eine Aussparung des zweiten Schnappverriegelungsmittels (13) in dem Schaft (21) vorgesehen ist und wobei die Vorsprünge (13) des ersten Schnappverriegelungsmittels (12) nach vorne gerichtete abgeschrägte Abschnitte umfassen, die auf den Schaft (21) ausgerichtet sind, um die Vorsprünge (13) beim Einführen des Schafts (21) zwischen die abgeschrägten Abschnitte nach und nach auseinanderzudrücken.

13. System, umfassend eine Vorrichtung (1) zur Selbstentnahme von Proben nach einem der vorhergehenden Ansprüche und einen verschließbaren Behälter (26) zum Lagern des Probensammelkörpers (3) nach der Verwendung.

## Revendications

1. Dispositif d'auto-échantillonnage (1) pour un fluide vaginal provenant du vagin d'une utilisatrice, ledit dispositif d'auto-échantillonnage (1) comprenant un premier corps allongé (2) destiné à être inséré dans le vagin de l'utilisatrice, et
un corps de collecte d'échantillon (3) pour collecter un échantillon de fluide vaginal de l'utilisatrice,
le corps de collecte d'échantillon (3) pouvant être fixé à une partie d'extrémité intérieure (4) du premier corps allongé (2) par un moyen de liaison libérable (5),
le premier corps allongé (2) étant configuré de telle sorte qu'il peut être actionné par l'utilisatrice pour collecter un échantillon de fluide dans le vagin avec le corps de collecte d'échantillon (3) fixé au premier corps (2),
ledit moyen de liaison libérable (5) comprenant un premier moyen d'interverrouillage (6) prévu sur l'un parmi le premier corps (2) et le corps de collecte d'échantillon (3), et un second moyen d'interverrouillage (7) prévu sur l'autre parmi le premier corps allongé (2) et le corps de collecte d'échantillon (3),
le premier moyen d'interverrouillage (6) étant pourvu d'un arbre (8) définissant un axe de rotation (9) sensiblement transversal à un axe longitudinal (10) du premier corps (2),
le second moyen d'interverrouillage (7) comprenant un premier trou ou évidement (11) configuré pour recevoir l'arbre (8) lors du raccord des premier (6) et second (7) moyens d'interverrouillage pour la rotation du corps de collecte d'échantillon (3) par rapport au premier corps (2) autour de l'axe de rotation (9) entre une première position (p1), dans laquelle l'axe longitudinal (18) du corps de collecte d'échantillon (3) est aligné avec l'axe longitudinal (10) du premier corps (2), et une seconde position (p2) dans laquelle l'axe longitudinal (18) du corps de collecte d'échantillon (3) est entraîné en rotation autour de l'axe de rotation (9) à l'écart de ladite première position (p1) d'une distance prédéterminée (D),
la rotation de l'arbre (8) par rapport au trou (11) exposant des profils projetés de forme différente, de sorte que, lorsqu'il se trouve dans la seconde position (p2), le profil projeté s'inscrit dans les limites d'un profil projeté correspondant exposé par un passage (22) du second moyen d'interverrouillage (7), et de telle sorte que, lorsqu'il ne se trouve pas dans la seconde position (p2), le profil projeté de l'arbre (8) ne s'inscrit pas dans les limites du profil projeté exposé par le passage (22),
les premier (6) et second (7) moyens d'interverrouillage étant en outre pourvus de premier (12) et second (13) moyens d'encliquetage correspondants respectifs configurés pour s'engager de manière libérable l'un avec l'autre (12), (13) lorsqu'ils se trouvent dans la première position (p1) pour empêcher une rotation relative entre les premier (6) et second (7) moyens d'interverrouillage lorsque les moyens d'encliquetage (12, 13) sont engagés,
les premier (6) et second (7) moyens d'interverrouillage étant configurés de telle sorte que, dans ladite seconde position (p2), les premier (6) et second (7) moyens d'interverrouillage peuvent être désengagés par un déplacement de l'arbre (8) hors du premier trou ou évidement (11) dans une direction de désengagement (19) prédéterminée, et l'arbre (8) et le premier trou ou évidement (11) étant configurés de telle sorte que, dans ladite première position (p1), le corps de collecte d'échantillon (3) est empêché de se désengager de la partie d'extrémité intérieure (4) du premier corps allongé (2) étant donné que le profil projeté de l'arbre ne s'inscrit pas dans les limites du profil projeté exposé par le passage.

2. Dispositif d'auto-échantillonnage (1) selon la revendication 1, l'arbre (8) étant prévu au niveau d'une première partie proximale (14) du premier moyen d'interverrouillage (6), le premier (12) des moyens d'encliquetage correspondants (12, 13) étant prévu au niveau d'une première partie distale (15) du premier moyen d'interverrouillage (6).

3. Dispositif d'auto-échantillonnage (1) selon la revendication 2, la première partie proximale (14) du premier moyen d'interverrouillage (6) étant adjacente au premier corps (2) ou au corps de collecte d'échantillon (3) sur lequel le premier moyen d'interverrouillage (6) est prévu, et la première partie distale étant plus éloignée du corps de collecte d'échantillon ou du premier corps sur lequel le premier moyen d'interverrouillage est prévu que la première partie proximale.

4. Dispositif d'auto-échantillonnage (1) selon l'une quelconque des revendications 1 à 3, le premier trou ou évidement (11) étant ouvert latéralement de telle sorte que l'arbre (8) est déplaçable latéralement hors du premier trou ou évidement (11) depuis ladite seconde position (p2) au moment du désengagement.

5. Dispositif d'auto-échantillonnage (1) selon la revendication 1, le premier moyen d'interverrouillage (6) comprenant un corps en fourche comprenant des branches opposées (16, 17) définissant ensemble un espace intermédiaire (20) s'étendant selon l'axe longitudinal (10) du premier corps (2),
ledit arbre (8) s'étendant d'une branche (16) à l'autre (17) en travers dudit espace intermédiaire (20), le second moyen d'interverrouillage (7) comprenant une tige allongée (21) adaptée pour s'ajuster dans l'espace intermédiaire (20) entre les branches (16, 17), et
ledit premier trou ou évidement (11) du second moyen d'interverrouillage (7) étant ouvert latéralement pour recevoir l'arbre (8).

6. Dispositif d'auto-échantillonnage (1) selon la revendication 5, l'arbre (8) étant prévu au niveau d'une première partie proximale (14) du premier moyen d'interverrouillage (6), un premier (12) des moyens d'encliquetage correspondants (12, 13) étant prévu au niveau d'une première partie distale (15) du premier moyen d'interverrouillage (6).

7. Dispositif d'auto-échantillonnage (1) selon l'une quelconque des revendications 5 ou 6, le premier trou ou évidement (11) étant ouvert latéralement dans une direction le long de la longueur du second moyen d'interverrouillage (7).

8. Dispositif d'auto-échantillonnage (1) selon la revendication 7,
l'arbre (8) étant principalement cylindrique avec au moins une partie évidée (24) le long de la longueur de l'arbre (8), et
le premier trou ou évidement (11) ayant une forme en section transversale avec une partie médiane circulaire (23) correspondant au diamètre principal de l'arbre (8),
ladite partie médiane (23) débouchant latéralement vers l'extérieur sous la forme du passage (22) d'une plus petite largeur inférieure au diamètre de l'arbre (8), et la ou les parties évidées (24) de l'arbre (8) définissant un profil projeté de l'arbre (8) dans un plan passant par l'axe longitudinal de l'arbre (8), ledit profil projeté étant plus petit que le passage (22) de telle sorte que l'arbre (8) est mobile à travers le passage (22) depuis ladite seconde position (p2).

9. Dispositif d'auto-échantillonnage (1) selon la revendication 8, le passage (22) s'élargissant vers l'extérieur en s'éloignant de la partie médiane circulaire (23).

10. Dispositif d'auto-échantillonnage (1) selon l'une quelconque des revendications 7 à 9, le premier trou ou évidement (11) comprenant une partie évidée auxiliaire (25) s'étendant vers l'intérieur à partir de la partie médiane circulaire (23) le long de la longueur du second moyen d'interverrouillage (7), en s'éloignant du passage (22) et en partie vers le second moyen d'encliquetage (13), la largeur de la partie évidée auxiliaire (25) étant inférieure au diamètre de la partie médiane (23).

11. Dispositif d'auto-échantillonnage (1) selon l'une quelconque des revendications 7 à 10, les moyens d'encliquetage correspondants (12, 13) étant prévus dans l'interface entre la tige (21) et les branches (16, 17).

12. Dispositif d'auto-échantillonnage (1) selon la revendication 11, des saillies (12) du premier moyen d'encliquetage (12) étant prévues sur les branches (16, 17), au moins un évidement du second moyen d'encliquetage (13) étant prévu dans la tige (21), et les saillies (13) du premier moyen d'encliquetage (12) comprenant des parties biseautées vers l'avant orientées pour que la tige (21) écarte progressivement les saillies (13) lors de l'insertion de la tige (21) entre les parties biseautées.

13. Système comprenant un dispositif d'auto-échantillonnage (1) selon l'une quelconque des revendications précédentes, un récipient scellable (26) étant destiné à stocker le corps de collecte d'échantillon (3) après utilisation.
